# EUROPEAN PATENT APPLICATION

(11) **EP 1 410 812 A1**
(43) Date of publication of application: **21.04.2004**
(21) Application number: 02292525.9
(22) Date of filing: 14.10.2002
(51) Int. Cl.: A61L 31/16

(54) **Functionalisation of stents with lipase**

(71) Applicant: EC (European Community), 2920 Luxembourg (LU)
(72) Inventor: McSweeney, Finbare Alain, 1049 Bruxelles (BE); Rossi, François, 21024 Biandronno (VA) (IT); Gribaldo, Laura, 28064 Carpignano Sesia (NO) (IT)
(74) Representative: Freylinger, Ernest T.

(57) **Abstract**

The invention relates to intravascular stents comprising in its inner surface an enzyme capable of catabolizing cholesterol and lipids, such as lipase, or cells that have been genetically modified to produce said enzyme, and to their use in the frame of the treatment or prevention of obstructive artheriosclerotic lesions in the coronary and peripheral blood vessels, or prevention of restenosis in intra coronaric stents.

## Description

The invention relates to intravascular stents comprising in its inner surface an enzyme capable of catabolizing cholesterol and lipids, such as lipase, or cells that have been genetically modified to produce said enzyme, and to their use in the frame of the treatment or prevention of obstructive artheriosclerotic lesions in the coronary and peripheral blood vessels, or prevention of restenosis in intra coronaric cells.

Coronary and peripheral angioplasty is routinely performed to treat obstructive aertheriosclerotic lesions in the coronary and peripheral blood vessels. Following balloon dilation of these blood vessels, 30-40% of patient undergo restenosis: Restenosis is the re-closure of a peripheral or coronary artery following trauma to that artery caused by efforts to open a stenosed portion of the artery, such as, for example, by balloon dilation, ablation atherectomy or laser treatment of the artery.

Restenosis is believed to be a natural healing reaction to the injury of the arterial wall. The healing reaction begins with the thrombotic mechanism at the site of the injury. The final result of the complex steps of the healing process can be intimal hyperplasia, the uncontrolled migration and proliferation of medial smooth muscle cells combined with their extracellular matrix production, until the artery is again stenosed or occluded. Thus, restenosis is characterized by both elastic recoil or chronic constriction of the vessel in addition to abnormal cell proliferation.

Currently restenosis must be treated with subsequent angioplasty procedures. In attempt to prevent restenosis, metallic intravascular stents have been permanently implanted in coronary or peripheral vessels. For example, U.S. 5.304.122 (Schwartz et al.) describes metal stents useful for treating restenosis after balloon angioplasty or other coronary interventional procedures. Preferred material for stents include metallic alloys such as Nitinol (currently preferred), stainless steel, and tantalum as well as different types of polymers.

The stent is typically inserted by catheter into a vascular lumen and expanded into contact with the diseased portion of the arterial wall, thereby providing mechanical support for the lumen. However, the aftermath of angioplasty is often problematic as the blood vessel can be re-close. In addition the stent itself can cause undesirable local thrombosis.

To address the problem of thrombosis, persons who receive stents must have extensive systemic treatment with anticoagulant drugs. Furthermore, other plaques can become unstable and rupture causing myocardial infarction and strokes. Wound repair of the blood vessel after balloon catheter dilation and stent placement involves proliferation of medical smooth muscle cells to form a thickened neointima. When a stent is placed at the site, the neointima grows over the stent. In about 30% of patients, this tissue remodelling process produces restenosis (i.e., narrowing) of the vessel, and a recurrence of related symptoms.

In order to prevent these problems, coating for stents have become an intense area of research. Stents coated with various compositions have been proposed. Several routes have been explored including coating of stents by isotropic or pyrolitic carbon (e.g. EP 556 940 A1 to Fischell or US 5163958 to Pinchuck), a polymeric coating having lubricious or anti coagulation properties (for instance US6159142 to Eckhard).

Other coatings with endothelial cells, or coating with drug release properties have also been proposed: For example, Dichek et al. (Circulation 1989, 80: 1347-1353) describe coating stainless steel with sheep endothelial cells that had undergone retrovirus-mediated gene transfer for either bacterial B-galactosidase of human tissue-type plasminogen activator. The stents were studied ex vivo in tissue culture dishes only. The feasibility of implanting the stents into arteries was not explored. Another patent with endothelial cells coatings is illustrated in WO 0001795 to Bowlin. This procedure of coating stents with cells is tedious, cumbersome and costly because cells have to be derived from a living organism.

Catheters or stents with attachments for administering a therapeutic agent have been described in U.S. patent Nos. 4.824.436 to Wolinsky, 4.832.688 to Sagae et al., and 5.254.089 to Wang.

Stents having a medicament-containing portion have been described in U.S. PATENTS No 5.419.760 to Narciso Jr., and 5.439.446 to Barry. U.S. Patent No 5.616.608 to Kinsella et al. describes a method of treating patients at risk of developing a vascular disease with an antimicrobial agent such as Taxo ® (paclitaxel), or a water soluble taxol derivate. U.S. Patent No 5.304.121 to Sahatjian describes a vascular catheter and balloon catheter for delivering an aqueous-mobile drug, where a portion of the exterior surface of the expandable balloon has a drug-impregnated hydrogel. U.S. Patent 5.716.981 to Hunter et al. describes a drug eluting stent coated with an anti-angiogenic composition having a polymeric carrier. U.S. Patent No. 5.591.227 to Dinh et al. describes a drug eluting stent coated a solid composite of a polymer and therapeutic substance in an adherent layer, and fibrin in an adherent layer of the composite. U.S. Patent No. 5.773.428 to Castelhano et al. and U.S. Patent No. 5.773.438 to Levy et al. describe certain chemical agent which have MMP-inhibiting properties. U.S. Patent No. 5.698.515 to Plate et al; describes certain insulin-containing polymer compositions.

Other methods of providing therapeutic substances to the vascular wall by means of stents have also been proposed. For example, WO 91/12779, entitled "Intraluminar Drug Eluting Prosthesis" and WO 90/13332, entitled "Stent With Sustained Drug Delivery", suggest coating stents with antiplatelet agents, anticoagulant agents, antimicrobial agents, anti-inflammatory agents, antimetabolic agents and other drugs to reduce the incidence of restenosis.

Similarly, U.S. 5.571.166 and 5.554.182 (both to Dinh et al.) describe intraluminal stents coated with fibrin and heparin. The stent is used to treat restenosis. Other examples include coating of stents by collagen (WO 9529647 to Buirge et al.) or protein C (WO 9949907 to De Bono et al.).

Other examples of patents include the composition of the layer with which the bioactive agents are included for controlled release. For instance, WO 9955396 to Chudzik describes the mixture of a bioactive agent in combination with layer compositions of poly butyl metacrylate and polyethylene co-vinyl acetate.

In the present invention, the stent is functionalised by the immobilisation of the enzyme on the device or by growth of cells that have been genetically modified to produce the enzyme catabolizing cholesterol and lipids.

These two treatments address the re-accumulation of lipids in the vein after the stent has been put in place. Before modification, the surface of the stents is covered by an underlayer in order to allow the cell growth.

Thus, the invention relates to an intravascular stent characterized in that it comprises in its inner surface an enzyme capable of catabolizing cholesterol and lipids, or cells that have been genetically modified to produce said enzyme.

The invention relates more particularly to intravascular stents as defined above, characterized in that said enzyme is chosen among lipoprotein lipase or the very low density lipoprotein (VLDL) receptor (VLDR).

Preferred intravascular stents according to the invention, are characterized in that the material constituting the stent is chosen among different metallic alloys such as stainless steel, shape memory alloys such as Ni, Ti based alloys of similar composition.

The invention also relates to intravascular stents as defined above, characterized in that the inner surface is covered by an underlayer capable to bind to the enzyme or to the genetically modified cells as mentioned above, and in this last case to allow the growth of said cells.

Advantageously, the underlayer mentioned above is an nitrogen rich layer such as polymers containing nitrogen and related chemical functionalities, and more particularly amorphous carbon nitrogen layer.

The invention also concerns intravascular stents as mentioned above, characterized in that the enzyme as defined above, is immobilized on the device surface by covalent binding with the polymer layer.

The invention also relates to intravascular stents as mentioned above, characterized in that the genetically modified cells as defined above, are bound to the layer, if necessary via a fibronectine coating.

The invention concerns more particularly intravascular stents as mentioned above, characterized in that the genetically modified cells are chosen among endothelial cells.

The invention relates more particularly to intravascular stents as defined above, characterized in that the genetically modified cells are chosen among human normal umbilical vein endothelial cells, or human autologous immortalized microvascular cells.

In a preferred embodiment, such intravascular stents as mentioned above, are characterized in that the endothelial cells are transformed with an AAV containing the sequence encoding the enzyme as defined above.

The invention also relates to the use of an intravascular stent as defined above, in the frame of the treatment or prevention of obstructive artheriosclerotic lesions in the coronary and peripheral blood vessels, or prevention of restenosis in intra coronaric stents.

The invention will be further illustrated in the following detailed description of stents according the invention.

### Part 1: preparation of underlayer

The cells are immobilized on the stent surface by preferential bonding on an nitrogen rich layer.

This layer is prepared from an amorphous carbon nitrogen layer (a-CN:H). The a-CN:H layer as a N/C ratio of 0.1 to 0.5 and hydrogen concentration between 2 at 50 at % , and is deposited on the device surface by different techniques such as plasma deposition from C and N containing gas sources, or by chemical transport from a graphite precursor.

The carbon can be produced by sputtering of a graphite target, or by a gaseous precursor containing carbon, such as methane, acetylene or other gases of this type. For instance, in the case of a solid carbon source, the carbon flux is obtained by DC or RF magnetron sputtering of a graphite target with an Argon/nitrogen/hydrogen mixture or a mixture of precursors containing these species (Ar + ammonia for instance).

Powers applied on the target are typically of the order of 500 to 1500W depending on the chamber and target size. The pressure for deposition can be between 1 to 100 mTorr, depending on the type of source used.

The concentration of nitrogen in the gas phase can be anything between 1.2 and 1.8g/cm3. The thickness of the films is lower than 1um and the films indicate different C-N bonds from IR absorption spectroscopy.

In the case of chemical transport reaction, a plasma source is used to produce atomic nitrogen, that reacts with a carbon mesh and produces radicals from which the film is grown. The pressure for deposition is typically in the 100 mTorr to the 10 torr range. The plasma source can be produce by a high frequency or microwave generator, and used to dissociate nitrogen and hydrogen to produce reactive species. These species react with the graphite mesh to produce CN, CH and NH radicals with condensate to form a film containing different concentrations of those components.

The advantage of the carbon nitrogen coating lies in the fact that a controllable density of NH group termination is achievable on the surface. Enclosed in the invention is the treatment by a plasma of H or NH containing gases of the carbon nitride surface after deposition in order to control the surface density of nitrogen and NH moieties on the surface.

The plasma is produced with a glow discharge in order to modify the surface composition and produce NH bonds to which the cells will be bound. The treatment is done in near post discharge of a plasma source, the gas discharge being any mixture of a rare gas such as Ar or He and N and H containing gas (such as N2/H2 or NH3) or derived compounds. The enzyme is immobilized on the device surface by covalent bonding with a polymer layer modified to present at its surface controlled concentration of amine or carboxylic groups to which the enzyme can be covalently bound.

This bonding is made by ionic bonding reaction, carboimide (water soluble carboimide/N-hydroxy succinimide) or reductive amination, all techniques already describes in the literature. In case of bonding between the cells and the surface, improvement can be obtained through a coating with fibronetic 100ug/ml.

### Part 2 : Preparation of the engineered cells

Lipoprotein lipase (LPL) plays a major role in the lipolysis of circulating lipoproteins. It functions in the rate-limiting hydrolysis of triacylglycerols (TGs) from circulating chylomicrons (CMs) and very low-density lipoprotein (VLDL), thereby releasing free fatty acids for cellular uptake and energy metabolism.

Gene therapy to deliver and express LPL protein may improve the lipid profile and reduce potential atherogenic risk from hypertriglyceridemia.

We propose to apply a strategy based on the ectopic expression of the LPL. We hypothesized that ectopic expression of LPL would reduce plasma TGs (triacylglycerols) and normalizing plasma lipoprotein metabolism. The expression of LPL in HUVEC cell line (human normal umbilical vein endothelial cells) obtained by transfection with an adeno-associated viral vector (AAV), would lead to a significant reduction of the formation of atherosclerosis.

Adeno-Associated virus (AAV) is a non-pathogenic virus that is widespread in the human population. Is nearly invisible to the human immune system and can infect human dividing and non-dividing cells. This is the only known mammalian virus that shows preferential integration into specific regions of the genome. AAV is naturally replication-deficient and normally requires coinfection with an unrelated helper virus, like adenovirus, to generate AAV virions. There are recent reports where AAV vectors carrying exogenous DNA were introduced into cultured mammalian cells and have been integrated into the cell genome via homologues recombination (Russell and Hirata, Nat Genet 1998 Apr;18(4):325-30).

The demonstration that AAV vector can undergo homologous recombination with single-copy chromosomal sequences, offers new hope that there is still room for further improvement in viral vector design.

Using the system, genes can be delivered into a human cell line and the protein can be expressed having all the necessary post-translation modifications and folding.

### 2.1 Cell line

HUVEC cell line (CRL-2480, American Type Culture Collection catalogue), human normal umbilical vein endothelial cells, will be used. The cells, grown in Ham's F12K medium with 2mM L-glutamine adjusted to contain 1.5 g/L sodium bicarbonate and supplemented with 0.1mg/ml heparin and 0.03 mg/ml endothelial cell growth supplement, fetal bovine serum 10%, are cultured at 37°C in a humidified atmosphere. A subcultivation ratio of 1:4 is recommended.

Alternatively, human autologous immortalized microvascular cells can be used.

### 2.2 Construction of the recombinant adeno-associated viral vector (AAV) and transfection of the cells

To generate HUVEC cell line overexpressing LPL, AAV Helper-Free-System (Stratagene, cat#240071) will be used. Adeno-associated vector expressing human LPL cDNA driven by cytomegalovirus (CMV) enhancer/chicken beta-globin promoter is constructed. The construct contains an expression cassette which include CMV-enhancer with a chicken beta-globin promoter, human LPL cDNA and simian virus 40 (SV40) polyadenylation signal, flanked with AAV ITRs in both 5' and 3' end.

The recombinant vector is contransfected into a packing cell line 293 (human embryonic kidney cell line) with the pAAV-RF and pHelper vector. pAAV-RF vector harbours the *cap* and *rep* gene which encode for the capsid and DNA replication proteins required to make the infectious virions. pHelper Vector carries the adenovirus genes (E2A, E4) required for inducing the lytic phase of AAV.

The cotransfection of all these plasmids will produce recombinant, replication-deficient virions. Lysates containing the virions are prepared and subject to three successive gradient of CsCl ultracentrifugation. Fraction containing the recombinant AAV (rAAV) are collected, pooled and dialyzed against PBS. The titer of virus in term of genome copies, are determined by a quantitative Southern blotting. Contamination of wild-type adenovirus is excluded by polymerase chain reaction. The virions are used to transfect HUVEC cell line that will overexpress the LPL enzyme.

### 2.3 Cells growth and adhesion to the stent

Tissue culture vessels used to propagate this cell line must be pre-coated with 0.1 % pig gelatin. The cells grow adherent. Alternatively, a coating with fibronectine 100ug/ml can be used.

The detection of transgene expression is done by immunofluorescence staining and Western blotting. The overexpression of LPL in HUVEC cells must be tested using increasing levels of lipoproteins added in the culture medium.

Once the cells have been transfected to produce LPL, the seeding on the stent can be done directly placing the stent covered with the underlayer described in part 1 above, in culture media with cells for two weeks, assessing the seeding efficiency by scanning electron microscopy.

### REFERENCES

1. J. Long Term Eff Med Implants 2000; 10(1-2):79-95
2. Exp Clin Endocrinol Diabetes 1997; 105 Suppl 2:35-7
3. J Biomater Sci Polym Ed 1998; 9(11):1117-35
4. Artif Cells Blood Substit Immobil Biotechnol 1996 Jan;24(1):51-63
5. J Biomed Mater Res 1995 Oct;29(10):1193-9
6. J Cell Sci 1986 Jun;82-263-80
7. Nat Genet, 1993, 4:27-34
8. Hum Gene Ther, 1997, 8:205-14
9. Hum Gene Ther, 2000, 11:21-32
10. Nat Genet, 1998, 18(4):325-30

## Claims

1. Intravascular stent **characterized in that** it comprises in its inner surface an enzyme capable of catabolizing cholesterol and lipids, or cells that have been genetically modified to produce said enzyme.

2. Intravascular stent according to claim 1, **characterized in that** said enzyme is chosen among lipoprotein lipase or the very low density lipoprotein (VLDL) receptor (VLDR).

3. Intravascular stent according to claim 1 or 2, **characterized in that** the material constituting the stent is chosen among different metallic alloys such as stainless steel, shape memory alloys such as Ni, Ti based alloys of similar composition.

4. Intravascular stent according to any of claims 1 to 4, **characterized in that** the inner surface is covered by an underlayer capable to bind to the enzyme or to the genetically modified cells as mentioned in claim 1, and in this last case to allow the said cells growth.

5. Intravascular stent according to claim 4, **characterized in that** the underlayer is an nitrogen rich layer such as polymers containing nitrogen and related chemical functionalities, and more particularly amorphous carbon nitrogen layer.

6. Intravascular stent according to claim 4 or 5, **characterized in that** the enzyme as defined in claim 1, is immobilized on the device surface by covalent binding with the polymer layer.

7. Intravascular stent according to claim 4 or 5, **characterized in that** the genetically modified cells as defined in claim 1, are bound to the layer, if necessary via a fibronectine coating.

8. Intravascular stent according to any one of claims 1 to 5 or 7, **characterized in that** the genetically modified cells are chosen among endothelial cells.

9. Intravascular stent according to claim 8, **characterized in that** the genetically modified cells are chosen among human normal umbilical vein endothelial cells, or human autologous immortalized microvascular cells.

10. Intravascular stent according to claim 8 or 9, **characterized in that** the endothelial cells are transformed with an adeno-associated viral vector (AAV) containing the sequence encoding the enzyme as defined in claim1 or 2.

11. Use of an intravascular stent according to any of claims 1 to 10, in the frame of the treatment or prevention of obstructive artheriosclerotic lesions in the coronary and peripheral blood vessels, or prevention of restenosis in intra coronaric stents.
